# FASCICULE DE BREVET EUROPEEN

(11) **EP 3 357 460 B1**
(45) Date de publication et mention de la délivrance du brevet: **15.04.2020**
(21) Numéro de dépôt: 18155176.3
(22) Date de dépôt: 05.02.2018
(51) Int. Cl.: A61F 5/01

(54) **ORTHÈSE DYNAMIQUE**
DYNAMISCHE ORTHESE
DYNAMIC ORTHOSIS

(30) Priorité: 06.02.2017 FR 1750970
(43) Date de publication de la demande: 08.08.2018
(73) Titulaire: Teyssedre, Hervé, 95660 Champagne sur Oise (FR)
(72) Inventeur: Teyssedre, Hervé, 95660 Champagne sur Oise (FR)
(74) Mandataire: Cabinet Camus Lebkiri

(56) Documents cités:
- EP-A1- 1 382 317
- WO-A1-2004/066890
- FR-A- 731 133
- FR-A1- 2 953 126
- FR-A1- 2 953 129
- SU-A1- 1 454 461
- US-A- 3 086 522

## Description

### DOMAINE TECHNIQUE

La présente invention concerne une orthèse dynamique de pied adaptée à l'aide à la marche ou à la rééducation d'un patient atteint d'une paralysie du pied ou bien des muscles ou nerfs releveurs. L'invention trouve des applications dans le domaine médical et, en particulier, dans le domaine de l'orthopédie.

### ETAT DE LA TECHNIQUE

Dans le domaine médical, les patients atteints de certaines affections musculaires ou neurologiques, telles que l'hémiplégie, la sclérose en plaques, les polynévrites, etc., peuvent avoir un pied ballant, c'est-à-dire que leur pointe de pied tombe naturellement vers le sol lorsque la jambe est relevée. Cet effet de pied ballant, appelé steppage du pied, est la conséquence d'une absence partielle ou totale de motricité musculaire rendant l'articulation de la cheville molle. Un patient souffrant d'un steppage du pied doit, pour se déplacer, lever exagérément la jambe de façon à ce que son pied n'accroche pas le sol.

Pour maintenir le pied du patient dans une position non-ballante, il est connu d'utiliser des orthèses rigides ou semi-rigides qui bloquent le pied dans une position sensiblement perpendiculaire à la jambe. Ces orthèses empêchent le pied de tomber mais bloquent également tous les mouvements de flexion du pied. Or, ces mouvements de flexion sont nécessaires à la marche. En conséquence, un patient équipé d'une orthèse rigide ou semi-rigide a une démarche asymétrique, de type claudication. En outre, de telles orthèses ne participent pas au travail de rééducation du pied du patient puisque le pied est totalement bloqué dans l'orthèse. Or, il est reconnu, dans le domaine médical, qu'il est important de faire travailler un membre, même lorsqu'il est paralysé, afin d'entretenir les muscles et articulations qui ne sont pas endommagées.

Il existe un autre type d'orthèse, moins rigide, qui permet la rééducation fonctionnelle et l'entretien des muscles et articulations, et en particulier des muscles releveurs. Cette orthèse comporte un moyen pour repousser le talon lorsque le pied est ballant, ou pour le tirer vers la jambe lorsque le pied est en appui. Une telle orthèse coopère avec le pied du patient, notamment avec l'articulation de la cheville et les articulations antérieures du pied. Il n'est donc pas nécessaire d'utiliser un organe externe d'articulation du pied, ce qui limite l'encombrement généré par l'orthèse.

Une telle orthèse comporte notamment un bracelet de jambe et une semelle de pied reliés ensemble par une structure de liaison. La structure de liaison comporte un vérin déporté sur l'arrière de la jambe et du pied. Le vérin est extensible longitudinalement pour faire pivoter la semelle autour de l'articulation de la cheville. Une telle orthèse est décrite notamment dans le brevet EP1382317, au nom du demandeur.

Le vérin est un vérin élastique qui comporte une extension axiale. Cette extension axiale est généralement une tige mobile montée dans un corps de vérin. Une extrémité inférieure de la tige de vérin est, par exemple, reliée à la semelle de pied au niveau du talon, tandis que le corps de vérin est relié au bracelet de jambe. Le vérin peut par exemple être un vérin pneumatique ou hydropneumatique. Il peut également s'agir d'un vérin mécanique, comportant un ressort hélicoïdal à l'intérieur du corps de vérin.

Le vérin est réglé de sorte que, lorsque le pied du patient est sensiblement perpendiculaire à l'orientation de la jambe, le vérin se trouve en semi-compression. Lorsque le patient soulève sa jambe du sol, la tige du vérin se déplace vers le bas, entraînant le talon de la semelle de pied. La semelle pivote, déplaçant vers le haut la partie antérieure du pied du patient, ce qui empêche les orteils dudit pied de frotter sur le sol au cours de la marche. Lorsque le patient pose à nouveau son talon sur le sol, la tige du vérin est poussée vers le haut. La partie antérieure du pied du patient pivote vers le bas et entre en contact avec le sol.

Dans ce type d'orthèse, la tige de vérin est généralement reliée à la semelle de pied par l'intermédiaire d'un système d'articulation comportant plusieurs degrés de liberté en rotation. Ce système d'articulation comporte généralement une articulation mécanique soudée sur la semelle de pied et faisant ainsi partie intégrante de la semelle de pied.

Toutefois, la liaison entre la semelle de pied et la tige de vérin, en particulier l'articulation mécanique, est soumise à une charge qui dépend notamment du poids du patient et de son activité, en particulier du nombre de pas effectués par le patient. Lorsque la charge est lourde, par exemple pour un patient dont la masse corporelle est supérieure à 80kg ou pour un patient très actif dont l'activité est supérieure à une activité normale de 6000 pas par jour, par exemple pour une activité de 8000 pas par jour ou plus, l'articulation mécanique peut être amenée à être remplacée une ou plusieurs fois pendant la durée de vie de l'orthèse afin d'éviter qu'elle ne se rompe.

Dans le cas d'une charge lourde, une orthèse telle que décrite précédemment doit être renvoyée au service après-vente du fabricant pour que soit dessoudée l'articulation mécanique défectueuse et soudée une nouvelle articulation mécanique, ce qui contraint le patient à se passer de l'orthèse pendant quelques temps.

Dans une autre orthèse, la structure de liaison peut relier ensemble le bracelet de jambe et la chaussure, le vérin de la structure de liaison étant directement reliée à la chaussure. Une telle orthèse est connue, par exemple, des documents FR 2 953 126 A1 et FR 2 953 129 A1, sur lesquels se fonde la préambule de la revendication 1. D'autres documents, comme les documents SU 1454461 A1, FR 731 133 A, WO 2004/066890 A1 et US 3 086 522 A, décrivent également des dispositifs orthopédiques dans lesquels le replacement de l'articulation mécanique n'est jamais évoqué.

### RESUME DE L'INVENTION

La présente invention propose une alternative aux orthèses décrites précédemment, dans laquelle l'articulation mécanique, démontable, peut être remplacée aisément sans nécessiter de renvoi chez le fabricant.

L'orthèse selon l'invention est définie dans la revendication 1. Elle comporte notamment une chaussure et un vérin avec une extension axiale reliée à la chaussure par l'intermédiaire d'un système d'articulation. Selon l'invention, ce système d'articulation comporte au moins deux éléments de rotation, mobiles l'un par rapport à l'autre, adaptés pour permettre une rotation d'un des éléments par rapport à l'autre, et un insert dans lequel est inséré, de façon amovible, un des éléments de rotation. Le premier élément de rotation, fixé dans l'insert, est un élément mâle. Le second élément de rotation, monté autour du premier élément de rotation, est un élément femelle.

De façon plus précise, l'invention concerne une orthèse dynamique comportant :
- une semelle de pied,
- une chaussure dans laquelle est montée la semelle de pied,
- un bracelet de jambe,
une structure de liaison déportée sur l'arrière de la jambe et du pied du patient et reliant ensemble le bracelet de jambe et la chaussure pour permettre à ladite chaussure de pivoter autour d'une articulation de cheville du patient, la structure de liaison comportant un vérin, une extension axiale dudit vérin et un système d'articulation monté entre une extrémité de l'extension axiale et la chaussure, le système d'articulation comportant :
▪ un insert solidaire de la chaussure,
▪ un premier élément de rotation amovible, muni d'une tête d'ancrage adaptée pour être insérée dans l'insert, et
▪ un second élément de rotation monté à l'extrémité de l'extension axiale, le premier et le second éléments de rotation étant assemblés pour permettre un mouvement rotatif de l'extension axiale par rapport à l'insert.

Cette orthèse a l'avantage de comporter un système d'articulation partiellement amovible permettant un échange aisé des premier et/ou second éléments de rotation lorsque ceux-ci sont endommagés. En outre, elle présente l'avantage de pouvoir être adaptée sur des chaussures spécifiques au regard des caractéristiques techniques d'une activité spécifique, par exemple sportive ou esthétique. Ainsi, cette orthèse permet au patient de vivre le plus normalement possible avec sa paralysie, notamment en portant des chaussures d'apparence « normale » qui ne ressemblent pas à des chaussures orthopédiques classiques.

Selon certains modes de réalisation, la tête d'ancrage du premier élément de rotation est filetée pour être vissée dans l'insert, ce qui donne au premier élément de rotation son caractère amovible.

Selon certains modes de réalisation, le système d'articulation comporte une platine de renfort dans laquelle est partiellement inséré l'insert. Cette platine de renfort augmente la surface de portance et de résistance de la structure de liaison de sorte à reprendre une partie au moins des efforts exercés par le système d'articulation sur l'insert.

Selon un ou plusieurs modes de réalisation de l'invention, la chaussure comporte un contrefort dans lequel est monté l'insert. Le contrefort assure alors la résistance et le maintien au pied du patient pour supporter l'énergie que le vérin génère en permanence lorsque la chaussure est montée autour du pied du patient.

Selon un ou plusieurs modes de réalisation de l'invention, la platine de renfort est intégrée dans le contrefort.

Selon certains modes de réalisation, le second élément de rotation est un élément de rotation femelle adapté pour être emboité autour du premier élément de rotation de sorte à former une articulation mécanique sphérique de type rotule offrant au système d'articulation plusieurs degrés de liberté en rotation.

Selon certains modes de réalisation, le premier élément de rotation comporte une bague de blocage assurant le blocage dudit premier élément de rotation dans l'insert.

Selon certains modes de réalisation, la chaussure comporte une doublure recouvrant le contrefort au moins à l'emplacement de l'insert, ce qui assure un confort optimal au patient.

Selon certains modes de réalisation, l'orthèse comporte des sangles (33) fixées bi-latéralement sur la chaussure (40), par exemple cousues entre la tige de la chaussure et sa doublure.

### BREVE DESCRIPTION DES FIGURES

D'autres avantages et caractéristiques de l'invention apparaîtront à la lecture de la description, illustrée par les figures dans lesquelles :
- La figure 1 représente une vue générale, latérale, d'un exemple d'orthèse selon l'invention ;
- La figure 2 représente une vue schématique, en coupe, d'un exemple du système d'articulation de l'orthèse selon l'invention,
- Les figures 3 et 4 représentent des vues en perspective d'exemples de premier et second éléments de rotation du système d'articulation de la figure 2,
- La figure 5 représente une vue en perspective des premier et second éléments de rotation des figures 3 et 4 lorsque ces éléments sont assemblés,
- La figure 6 représente une vue latérale, en coupe, d'un exemple d'insert du système d'articulation de la figure 2, et
- La figure 7 représente une vue en perspective d'une goupille de verrouillage, verrouillant le premier et le second éléments de rotation.

### DESCRIPTION DETAILLEE D'AU MOINS UN MODE DE REALISATION

Un exemple de réalisation d'une orthèse avec un système d'articulation partiellement démontable est décrit en détail ci-après, en référence aux dessins annexés. Cet exemple illustre les caractéristiques et les avantages de l'invention. Il est toutefois rappelé que l'invention ne se limite pas à cet exemple.

Sur les figures, les éléments identiques sont repérés par des références identiques. Pour des questions de lisibilité des figures, les échelles de taille entre éléments représentés ne sont pas respectées.

Dans la description qui suit, il est fait référence à des dimensions latérales et longitudinales, les dimensions longitudinales étant considérées suivant un axe X de position du pied du patient et les dimensions latérales étant considérées suivant un axe Z, perpendiculaire à l'axe X et à un axe Y d'allongement de la jambe du patient.

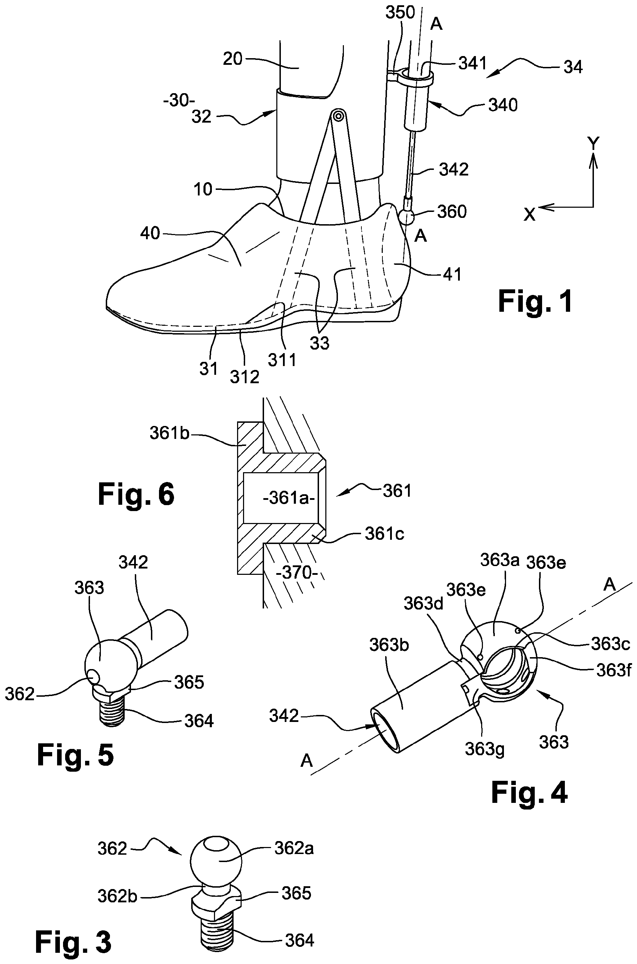

La figure 1 représente une orthèse dynamique 30 conforme à ou plusieurs modes de réalisation de l'invention. Cette orthèse 30 est montrée portée par un patient en station debout. L'orthèse 30 comporte une chaussure 40 adaptée pour recevoir le pied d'un patient. Elle comporte aussi un bracelet de jambe 32 destiné à être placé autour de la jambe 20 du patient. Le bracelet 32 peut prendre plusieurs formes connues de l'état de la technique. Par exemple, le bracelet 32 peut être d'une seule pièce, comme montré sur la figure 1. En variante, le bracelet 32 peut comporter deux parties enserrant deux zones différentes de la jambe 20, par exemple la cheville et le mollet, les deux zones étant reliées entre elles de façon solidaire.

L'orthèse 30 comporte également une semelle de pied 31. Cette semelle de pied 31 a une surface 311 en contact avec le pied du patient et une sous-face 312 destinée à être en contact avec la semelle intérieure de la chaussure 40 appelée aussi première de montage. La semelle de pied 31 est solidaire de la chaussure 40. Dans une variante, la semelle de pied 31 est déposée dans la chaussure 40 et maintenue par pression entre le pied du patient et la semelle intérieure de ladite chaussure. Dans une autre variante, la semelle de pied 31 est fixée dans la chaussure 40 par tous moyens de fixation connus dans les domaines de l'orthopédie et/ou de la cordonnerie.

Dans certains modes de réalisation, la semelle de pied 31 est une semelle intérieure amovible, appelée aussi première de propreté. Le pied du patient est alors soutenu par la chaussure elle-même dont la forme intérieure est adaptée à l'anatomie du pied du patient. La semelle de pied 31 peut comporter une plaquette dont la surface est plane ou, au contraire, dont la surface a une forme non plane, par exemple complémentaire de la voûte plantaire du patient. Dans d'autres modes de réalisation, la semelle de pied est une semelle dite « orthopédique », adaptée à l'anatomie du pied du patient. La semelle de pied 31 est alors montée à l'intérieur de la chaussure 40 pour soutenir au moins en partie le pied du patient. Cette semelle de pied 31 peut alors comporter une coque anatomique adaptée au pied 10 du patient.

Si l'on considère que la position du pied 10 est parallèle à l'axe X de position du pied, la longueur de la semelle de pied 31 selon cet axe X est égale à au moins 70% de la longueur du pied. Autrement dit, la semelle de pied 31 s'étend au moins du calcanéum au métatarse du pied, c'est-à-dire du talon jusqu'aux articulations des orteils. Elle peut s'étendre au-delà du métatarse, par exemple jusqu'aux orteils.

Selon un mode de réalisation, représenté sur la figure 1, la semelle de pied 31 est insérée à l'intérieur d'une chaussure 40. Elle présente alors, de préférence, une épaisseur faible, par exemple de 2 à 5mm. La chaussure 40 peut être une chaussure standard de formes et dimensions adaptées à la semelle de pied 31 ou une chaussure orthopédique adaptée au pied du patient. Quelle soit standard ou orthopédique, la chaussure 40 est équipée d'un contrefort 41 qui rigidifie la chaussure pour empêcher le pied de la déformer. Ce contrefort 41, réalisé par exemple dans une thermorésine, un thermoplastique, un carbone ou un cellulose, avec un galbe adapté à la chaussure, est relié au bracelet de jambe 32 par l'intermédiaire de la structure de liaison 34.

La structure de liaison 34 comporte un vérin mécanique 340, par exemple un vérin élastique, déporté sur l'arrière de la jambe 20 et du pied 10 par une pièce de fixation 350. Le vérin 340 comporte un corps de vérin 341 et une extension axiale 342 apte à coulisser à l'intérieur du corps de vérin 341. L'extension axiale 342, appelée aussi tige de vérin, comporte une extrémité supérieure montée mobile à l'intérieur du corps de vérin 341 et une extrémité inférieure fixée à un système d'articulation 360. La tige de vérin 342 s'étend suivant un axe AA.

La pièce de fixation 350 est fixée d'une part au bracelet de jambe 32 et d'autre part au corps du vérin 341 de façon à maintenir ledit vérin 340 suivant l'axe AA qui est sensiblement parallèle à l'axe Y de la jambe 20. La pièce de fixation 350 présente une ouverture permettant au vérin d'être mobile uniquement dans un mouvement d'arrière en avant, c'est-à-dire lorsque le pied, indépendant de la jambe, à la marche, se pose à plat au sol, et que la jambe n'a pas encore entamé son avancée de verticalisation au-dessus dudit pied portant. Le jeu dans l'ouverture de la pièce de fixation 350 permet d'éviter toute contrainte de cisaillement sur le vérin.

Le vérin 340 est extensible suivant l'axe AA qui est sensiblement parallèle à l'axe Y de la jambe. Le coulissement de la tige de vérin 342 dans le corps de vérin 341 entraîne le pivotement de la semelle 31 autour de l'articulation de la cheville du patient. Dans certains modes de réalisation, le vérin 342 est réglé en semi-compression lorsque la jambe 20 est perpendiculaire au pied 10 du patient. Ainsi, lorsque le patient soulève son pied du sol pour faire un pas, la tige de vérin 342 coulisse vers le bas (en direction du sol) et la semelle 31 pivote de manière à écarter du sol la pointe avant du pied. Au contraire, lorsque la tige de vérin 342 se déplace vers le haut (en direction de la cuisse du patient), elle entraine l'arrière de la semelle de pied 31, ce qui permet à l'avant du pied du patient de pivoter en direction du sol.

Dans certains modes de réalisation, l'orthèse 30 peut comporter des sangles 33 reliées entre la semelle de pied 31 et le bracelet de jambe 32 ou entre la chaussure 40 et le bracelet de jambe 32. Dans l'exemple de la figure 1, deux sangles 33 sont fixées sur la chaussure 40, de part et d'autre de ladite chaussure. Elles peuvent, par exemple être cousues bi-latéralement entre la tige de la chaussure et sa doublure. Les deux sangles peuvent, par exemple, être positionnées latéralement de chaque côté du pied 10, l'une reliant l'avant de la voute plantaire au bracelet de jambe 32, l'autre reliant le talon au bracelet de jambe 32. Une telle répartition des sangles permet une transmission optimale de la force fournie par le vérin 340. Ces sangles 33 ont notamment pour fonction de limiter les angles de torsion du pied 10 autour d'un axe parallèle à l'axe X, qui peuvent être à l'origine d'entorses.

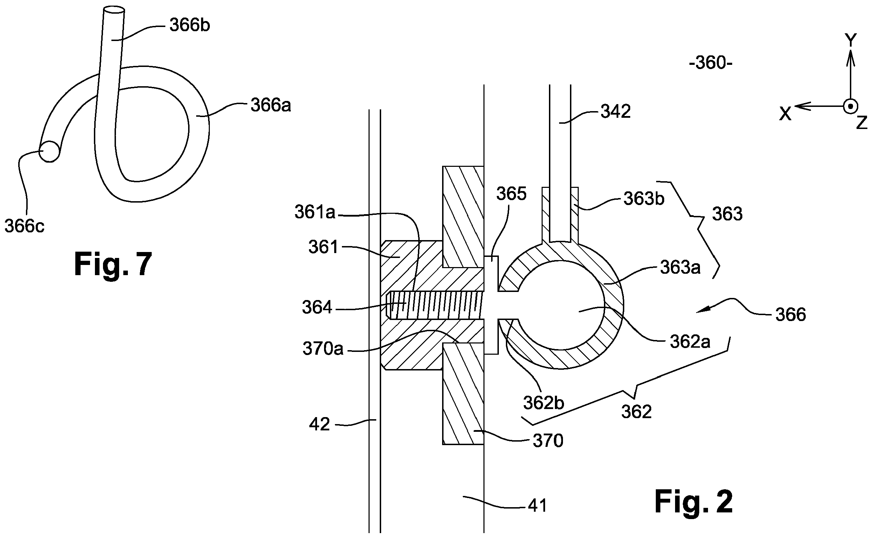

Le système d'articulation 360, dont un exemple est représenté sur la figure 2, comporte un insert 361 ainsi qu'un premier et un second éléments de rotation, respectivement 362 et 363. Le premier et le second éléments de rotation 362, 363 forment ensemble une articulation mécanique, démontable, qui comporte plusieurs degrés de liberté en rotation.

Le premier élément de rotation 362 comporte une tête d'ancrage 364 apte à être insérée dans l'insert 361. La tête d'ancrage 364 comporte une dimension longitudinale dans la direction de l'axe X et une dimension latérale dans la direction de l'axe Z, perpendiculaire aux axes X et Y. La dimension longitudinale est choisie de façon à assurer le maintien du premier élément de rotation 362 à l'intérieur de l'insert 361. Elle peut être, par exemple, de l'ordre de 4 à 8mm. La dimension latérale est déterminée de façon à ce que la tête d'ancrage soit suffisamment solide pour limiter les risques de rupture du système d'articulation. Selon certains modes de réalisation, la tête d'ancrage 364 est formée d'une tige filetée, par exemple d'un filetage métrique variant entre M5 ou M6. En variante, cette tête d'ancrage 364 peut comporter tout autre moyen de fixation amovible, c'est-à-dire tout moyen permettant au premier élément de rotation 362 d'être solidarisé avec l'insert tout en étant amovible.

L'insert 361 est une pièce, par exemple en métal, en alliage inoxydable ou en résine synthétique, solidaire de la chaussure 40, et en particulier du contrefort 41, qui est munie d'un orifice non traversant 361a adapté pour recevoir au moins en partie la tête d'ancrage 364 du premier élément de rotation 362. Dans les modes de réalisation où la tête d'ancrage 364 comporte une tige filetée, les parois de l'orifice non traversant 361a de l'insert 361 comportent un filetage dont le pas est adapté au pas de vis de la tête d'ancrage 364.

Le second élément de rotation 363 est fixé à l'extrémité inférieure de la tige de vérin 342 et relie ladite tige de vérin au premier élément de rotation 362. Le premier et le second éléments de rotation 362, 363 peuvent être assemblés directement l'un avec l'autre et former, par exemple, une liaison de type rotule. Ils peuvent également être reliés indirectement par l'intermédiaire d'un ou plusieurs autres éléments et former, par exemple, une articulation de type cardan.

Dans le mode de réalisation représenté sur les figures 2 - 5, le premier et le second éléments de rotation 362, 363 sont des éléments sphériques formant ensemble une liaison de type rotule 366. Le premier élément de rotation 362 peut être un élément de rotule mâle ; le second élément de rotation 363 peut être un élément de rotule femelle. Comme montré sur la figure 3, l'élément de rotule mâle 362 peut comporter un noyau sphérique 362a, par exemple en acier ou en alliage inoxydable. Le noyau sphérique peut avoir, par exemple, un diamètre de 8mm. L'élément de rotule femelle 363 peut comporter, comme montré sur la figure 4, une tête sphérique 363a entourant au moins partiellement le noyau sphérique 362a, comme montré sur la figure 5. La tête sphérique 363a, réalisée dans un acier ou un alliage inoxydable, par exemple identique à celui du noyau sphérique 362a, peut être clipsée sur le noyau sphérique 362a.

Dans les exemples des figures 2 et 4, la tête sphérique 363a est une sphère creuse, par exemple de diamètre 10mm, ouverte le long de la corde 363c. Le second élément de rotation 363 comporte un bras de fixation 363b accolé à la tête sphérique 363a dans le prolongement de la corde 363c et adapté pour être fixé à l'extrémité inférieure de la tige de vérin 342. Dans une variante représentée sur la figure 2, le bras de fixation est tubulaire et reçoit, en son sein, l'extrémité inférieure de la tige de vérin 342. Dans cette variante, l'intérieur du bras de fixation forme un tunnel fileté, par exemple de filetage M6, qui reçoit l'extrémité inférieure filetée (par exemple avec un filetage M5) de la tige de vérin 342. Dans une autre variante, le bras de fixation 363b peut être monté à l'intérieur de la tige de vérin 342.

Le bras de fixation 363b peut comporter, comme montré sur la figure 4, une gorge annulaire 363d destinée à recevoir une goupille de verrouillage 366 assurant le verrouillage du second élément de rotation 363 sur le premier élément de rotation 362. Dans ce cas, la tête sphérique 363a comporte deux trous excentrés 363e, alignés, adaptés pour être traversés par la goupille de verrouillage 366. Les deux trous excentrés 363e sont des trous traversants usinés dans la tête sphérique 363a suivant une droite parallèle à l'axe AA passant par la tige de vérin 342.

La goupille de verrouillage 366, dont un exemple est représenté schématiquement sur la figure 7, est un fil métallique ayant une forme d'anneau ouvert 366a muni, à une de ses extrémités, d'une tige rectiligne 366b formant un angle sensiblement droit avec le plan de l'anneau. Le fil métallique est réalisé dans un métal ou un alliage suffisamment résistant pour que la tige rectiligne 366b ne se déforme pas sous l'effet des rotations du second élément de rotation autour du premier élément de rotation et suffisamment élastique pour permettre à un opérateur d'exercer une traction sur l'anneau ouvert 366a sans déformer ledit anneau. Ainsi, lors du montage du second élément de rotation 363 sur le premier élément de rotation 362, une fois le second élément de rotation 363 emboité autour du premier élément de rotation 362, la tige rectiligne 366b peut être engagée dans le trou excentré le plus proche de la gorge annulaire 363d puis dans le trou excentré le plus éloigné de ladite gorge annulaire ; l'anneau ouvert 366a peut ensuite être positionné dans la gorge annulaire 363d de façon à verrouiller le premier élément de rotation 362 à l'intérieur du second élément de rotation 363.

Dans une variante, l'extrémité libre de la goupille de verrouillage 366 peut comporter une bille de préhension 366c permettant, notamment, d'attraper la goupille de verrouillage pour déverrouiller le second élément de rotation 363, lors d'un changement du premier et/ou du second élément de rotation.

Dans les exemples des figures 2 et 3, le noyau sphérique 362a du premier élément de rotation 362 est une sphère, pleine ou creuse, fixée indirectement sur la tête d'ancrage 364, par l'intermédiaire d'une bague d'emboitement 362b et d'une bague de blocage 365. La bague d'emboitement 362b est dimensionnée pour recevoir la tête sphérique 363a. Autrement dit, la bague d'emboitement 362b, positionnée entre la tête d'ancrage 364 et le noyau sphérique 362a, a des dimensions latérales adaptées aux dimensions de l'ouverture le long de la corde 363c. La bague de blocage 365 est adaptée pour bloquer l'élément de rotule mâle 362 dans l'insert 361. La bague de blocage 365 peut être un élément fixe, de dimensions latérales supérieures aux dimensions latérales de la tête d'ancrage 364 et de la bague d'emboitement 362b, positionné à demeure entre ladite tête d'ancrage 364 et la bague d'emboitement 362b de sorte que, non seulement ladite bague de blocage 365 bloque la tête d'ancrage 364 dans l'insert 361, mais aussi elle délimite la dimension longitudinale de la bague d'emboitement 362b accueillant la tête sphérique 363a.

Dans un mode de réalisation du second élément de rotation 363, ledit second élément de rotation 363 comporte des usinages réalisés dans le voisinage de la corde 363c. Par exemple, un premier usinage 363g peut être réalisé à la jonction entre la corde 363c et le bras de fixation 363b et un second usinage 363f peut être réalisé sur la corde 363c transversalement au premier usinage. Ces premier et second usinages 363g, 363f ont des formes et des dimensions adaptées pour permettre au second élément de rotation 363 de s'imbriquer au moins partiellement autour de la bague de blocage 365 du premier élément de rotation 362. De cette façon, le second élément de rotation 363 peut bouger avec une large amplitude autour du premier élément de rotation 362, c'est-à-dire selon plusieurs degrés de liberté, ce qui permet une grande flexion et une grande extension du pied du patient.

Dans le mode de réalisation où la semelle de pied 31 est insérée à l'intérieur de la chaussure 40, l'insert 361 est positionné à l'intérieur de la chaussure et fixé sur une platine de renfort 370 aménagée dans le contrefort 41 de ladite chaussure. Autrement dit, le contrefort 41 qui est fixé à l'intérieur de la chaussure 40 à l'endroit et sur les côtés de l'arrière du pied du patient, incorpore une platine de renfort 370 assurant une consolidation de l'insert 361. Ainsi, la platine de renfort 370 reprend, au moins en partie, les efforts exercés par la tête d'ancrage 364 sur l'insert 361. La platine de renfort 370 peut être fixée sur le contrefort 41 de la chaussure par un ou plusieurs moyens de fixation tels que des rivets ou intégrée dans le contrefort 41. La platine de renfort peut également être intégrée dans le matériau du contrefort 41 puis fixée par rivet ou sertissage sur ledit contrefort.

Cette platine de renfort 370 est une plaquette d'une épaisseur d'environ 1,5 à 3 mm, réalisée par exemple en métal ou en alliage inoxydable, et intégrée et/ou fixée sur le contrefort 41. La platine de renfort 370 est galbée, avec un galbe adapté à l'arrière du pied du patient, c'est-à-dire à la forme du pied entre le talon et la cheville.

Dans les modes de réalisation où la semelle de pied est insérée à l'intérieur de la chaussure, l'insert 361 est partiellement inséré dans la platine de renfort 370, ce qui augmente la surface de portance et renforce la liaison entre les éléments de rotation 362, 363 et la chaussure 40. Pour cela, comme montré sur la figure 6, l'insert 361 peut comprendre une embase 361 b destinée à être en appui contre la platine de renfort 370 et une couronne 361c destinée à s'insérer dans un orifice 370a de la platine de renfort. Cette couronne 361c peut comporter, sur sa paroi externe, un filetage lui permettant d'être vissée dans l'orifice 370a de la platine de renfort. La bague de blocage 365 est alors bloquée, via le contrefort 41, sur la platine de renfort 370. Dans les modes de réalisation où la platine de renfort 370 est fixée par rivets sur le contrefort, ladite platine de renfort comporte un ou deux trous (non représentés sur les figures) adaptés pour recevoir chacun un rivet. Ces trous sont de préférence alignés avec l'orifice 370a suivant l'axe Y.

Le contrefort 41 est alors muni d'un orifice non traversant destiné à recevoir la platine de renfort 370 et d'un orifice traversant destiné à recevoir l'insert 361. L'insert 361, qui est fixé sur la platine de renfort 370 par soudage ou vissage, reçoit la tête d'ancrage 364 du premier élément de rotation 362, lui-même placé en extérieur de chaussure. Le contrefort 41 de l'orthèse selon l'invention peut être un contrefort spécifique monté avec l'insert 361 et la platine de renfort 370 à l'intérieur de la chaussure, à la place du contrefort d'origine de ladite chaussure. Au contraire, le contrefort 41 peut être le contrefort d'origine de la chaussure qui est aménagé spécifiquement pour recevoir l'insert 361 et la platine de renfort 370.

Quel que soit le mode de réalisation, l'insert 361 a une profondeur relativement fine, sensiblement égale à l'épaisseur du contrefort 41, de quelques millimètres, par exemple 5 à 12mm] et une surface extérieure plane avec des angles biseautés de sorte à ne pas nuire au confort du pied du patient. En extérieur de chaussure, seul l'insert 361 est visible dans l'orifice traversant du contrefort 41.

Dans certains modes de réalisation, comme celui où la semelle de pied 31 est insérée à l'intérieur de la chaussure 40, une doublure 42 peut recouvrir l'intérieur de la chaussure 40, le long du contrefort 41. Cette doublure 42 peut recouvrir la totalité du contrefort 41 ou uniquement la zone du contrefort contenant l'insert 361. Une telle doublure assure un confort optimum au pied du patient dans l'orthèse.

Quel que soit le mode de réalisation décrit précédemment, les éléments de rotation 362, 363 peuvent aisément être échangés. Il suffit à un utilisateur de retirer, par exemple par dévissage, les éléments de rotation 362 et 363 en place, notamment lorsqu'ils sont endommagés, et de les remplacer par des éléments de rotation 362, 363 neufs. Le remplacement des éléments de rotation 362, 363 peut donc être effectué par le fournisseur de la semelle de pied, ou le patient lui-même, sans qu'il ne soit nécessaire de renvoyer la semelle de pied ou la chaussure équipée de la semelle de pied au service après-vente du fabriquant.

## Revendications

1. Orthèse dynamique (30) comportant :
- une semelle de pied (31),
- une chaussure (40) dans laquelle est montée la semelle de pied (31),
- un bracelet de jambe (32),
- une structure de liaison (34) déportée sur l'arrière de la jambe et du pied du patient et reliant ensemble le bracelet de jambe (32) et la chaussure (40) pour permettre à ladite chaussure de pivoter autour d'une articulation de cheville du patient, la structure de liaison (34) comportant un vérin (340), une extension axiale (342) dudit vérin et un système d'articulation (360) monté entre une extrémité de l'extension axiale et la chaussure,
**caractérisée en ce que** le système d'articulation (360) comporte :
▪ un insert (361) solidaire de la chaussure (40),
▪ un premier élément de rotation (362) amovible, muni d'une tête d'ancrage (364) adaptée pour être insérée dans l'insert (361), et
▪ un second élément de rotation (363) monté à l'extrémité de l'extension axiale (342), le premier et le second éléments de rotation étant assemblés pour permettre un mouvement rotatif de l'extension axiale par rapport à l'insert.

2. Orthèse selon la revendication 1, **caractérisée en ce que** la tête d'ancrage (364) du premier élément de rotation est filetée pour être vissée dans l'insert (361).

3. Orthèse selon la revendication 1 ou 2, **caractérisée en ce que** le système d'articulation (360) comporte une platine de renfort (370) dans laquelle est partiellement inséré l'insert.

4. Orthèse selon l'une quelconque des revendications 1 à 3, **caractérisée en ce que** la chaussure comporte un contrefort (41) dans lequel est monté l'insert (361).

5. Orthèse selon les revendications 3 et 4, **caractérisée en ce que** la platine de renfort (370) est intégrée dans le contrefort (41).

6. Orthèse selon l'une quelconque des revendications 1 à 5, **caractérisée en ce que** le second élément de rotation (363) est un élément de rotation femelle adapté pour être emboité autour du premier élément de rotation (362) de sorte à former une rotule.

7. Orthèse selon l'une quelconque des revendications 1 à 6, **caractérisée en ce que** le premier élément de rotation (362) comporte une bague de blocage (365) assurant le blocage dudit premier élément de rotation dans l'insert (361).

8. Orthèse selon l'une quelconque des revendications 4 à 7, **caractérisée en ce que** la chaussure (40) comporte une doublure (42) recouvrant le contrefort (41) au moins à l'emplacement de l'insert.

9. Orthèse selon l'une quelconque des revendications 1 à 8, **caractérisée en ce qu'**elle comporte des sangles (33) fixées bi-latéralement sur la chaussure (40).

## Patentansprüche

1. Dynamische Orthese (30), umfassend:
- eine Fußsohle (31),
- einen Schuh (40), in dem die Fußsohle (31) montiert ist,
- eine Beinmanschette (32),
- eine Verbindungsstruktur (34), die an der Rückseite des Beins und des Fußes des Patienten versetzt ist und die Beinmanschette (32) und den Schuh (40) miteinander verbindet, um dem genannten Schuh das Schwenken um ein Fußgelenk des Patienten zu erlauben, wobei die Verbindungsstruktur (34), einen Zylinder, eine axiale Verlängerung (342) des genannten Zylinders und ein Gelenksystem (360) umfasst, das zwischen einem Ende des axialen Endes und dem Schuh montiert ist,
**dadurch gekennzeichnet, dass** das Gelenksystem (360) umfasst:
▪ einen Einsatz (361), der fest mit dem Schuh (40) verbunden ist,
▪ ein erstes abnehmbares Drehelement (362), das mit einem Verankerungskopf (364) versehen ist, der geeignet ist, um in dem Einsatz (361) eingefügt zu sein, und
▪ ein zweites Drehelement (363), das am Ende der axialen Verlängerung (342) montiert ist, wobei das erste und das zweite Drehelement zusammengebaut sind, um eine Drehbewegung der axialen Verlängerung im Verhältnis zum Einsatz zu erlauben.

2. Orthese gemäß Anspruch 1, **dadurch gekennzeichnet, dass** der Verankerungskopf (364) des ersten Drehelements mit einem Gewinde versehen ist, um in den Einsatz (361) geschraubt zu sein.

3. Orthese gemäß Anspruch 1 oder 2, **dadurch gekennzeichnet, dass** das Gelenksystem (360) eine Verstärkungsplatine (370) umfasst, in der der Einsatz teilweise eingefügt ist.

4. Orthese gemäß irgendeinem der Ansprüche 1 bis 3, **dadurch gekennzeichnet, dass** der Schuh eine Hinterkappe (41) umfasst, in der der Einsatz (361) montiert ist.

5. Orthese gemäß irgendeinem der Ansprüche 3 bis 4, **dadurch gekennzeichnet, dass** die Verstärkungsplatine (370) in die Hinterkappe (41) integriert ist.

6. Orthese gemäß irgendeinem der Ansprüche 1 bis 5, **dadurch gekennzeichnet, dass** das zweite Drehelement (363) ein weibliches Drehelement ist, das geeignet ist, um um das erste Drehelement (362) derart eingepasst zu sein, dass ein Kugelgelenkt geformt ist.

7. Orthese gemäß irgendeinem der Ansprüche 1 bis 6, **dadurch gekennzeichnet, dass** das erste Drehelement (362) einen Feststellring (365) umfasst, der das Feststellen des ersten Drehelements in dem Einsatz (361) gewährleistet.

8. Orthese gemäß irgendeinem der Ansprüche 4 bis 7, **dadurch gekennzeichnet, dass** der Schuh (40) ein Futter (42) umfasst, das die Hinterkappe (41) wenigstens an der Stelle des Einsatzes abdeckt.

9. Orthese gemäß irgendeinem der Ansprüche 1 bis 8, **dadurch gekennzeichnet, dass** sie Bänder (33) umfasst, die beidseitig am Schuh (40) befestigt sind.

## Claims

1. A dynamic orthosis (30) including:
- a foot sole (31),
- a shoe (40) in which the foot sole (31) is mounted,
- a leg cuff (32),
- a connecting structure (34) offset to the back of the patient's leg and foot and connecting the leg cuff (32) and the shoe (40) together to enable said shoe to pivot around the patient's ankle joint, the connecting structure (34) including an actuator (340), an axial extension (342) of said actuator and a hinging system (360) mounted between an end of the axial extension and the shoe,
**characterised in that** the hinging system (360) includes:
▪ an insert (361) integral with the shoe (40),
▪ a first removable rotating element (362), fitted with an anchoring head (364) adapted to be inserted into the insert (361), and
▪ a second rotating element (363) mounted at the end of the axial extension (342), the first and second rotating elements being assembled to allow rotational motion of the axial extension relative to the insert.

2. The orthosis according to claim 1, **characterised in that** the anchoring head (364) of the first rotating element is threaded to be screwed in the insert (361).

3. The orthosis according to claim 1 or 2, **characterised in that** the hinging system (360) includes a reinforcing plate (370) into which the insert is partially inserted.

4. The orthosis according to any of claims 1 to 3, **characterised in that** the shoe includes a stiffener (41) in which the insert (361) is mounted.

5. The orthosis according to claims 3 and 4, **characterised in that** the reinforcing plate (370) is integrated into the stiffener (41).

6. The orthosis according to any of claims 1 to 5, **characterised in that** the second rotating element (363) is a female rotating element adapted to be snap-fit around the first rotating element (362) so as to form a ball joint.

7. The orthosis according to any of claims 1 to 6, **characterised in that** the first rotating element (362) includes a locking ring (365) ensuring locking of said first rotating element into the insert (361).

8. The orthosis according to any of claims 4 to 7, **characterised in that** the shoe (40) includes a lining (42) covering the stiffener (41) at least at the location of the insert.

9. The orthosis according to any of claims 1 to 8, **characterised in that** it includes straps (33) bilaterally attached to the shoe (40).
